# EUROPEAN PATENT APPLICATION

(11) **EP 3 369 709 A1**
(43) Date of publication of application: **05.09.2018**
(21) Application number: 18159493.8
(22) Date of filing: 01.03.2018
(51) Int. Cl.: C02F 1/32, C02F 1/36, B08B 9/032, B08B 9/08, C02F 1/00

(54) **TARGETED CLEANING IN WATER SYSTEM COMPONENTS**

(30) Priority: 01.03.2017 US 201715446835
(71) Applicant: Goodrich Corporation, Charlotte, NC 28217-4578 (US)
(72) Inventor: SCHOMER, Samual Steven Riczo, Akron, Ohio 44308 (US)
(74) Representative: Dehns

(57) **Abstract**

A self-cleaning component assembly includes a fluid containment vessel (14), an ultrasonic transducer (16) fixed to a wall of the fluid containment vessel, and a germicidal light source (20) fixed to the component such that germicidal light is directed to an internal cavity (24) of the fluid containment vessel.

## Description

### BACKGROUND

The present invention relates to fluid systems, and more particularly to the removal of biofilms and scale from fluid system components.

Biofilms and mineral scale can occur in fluid system components and can lead to clogging, corrosion, and a reduction in performance. Current methodology for removing biofilms and scale includes chemical treatment, physical cleaning, often done by hand or requiring components to be removed from the system, and replacement of components. Such methods can be time-intensive and can require the fluid system to be taken off-line for extended periods of time. In some situations, taking the fluid system off-line can require a shutdown of the entire system in which the fluid system is operating. For instance, cleaning potable water system components in an aircraft can require grounding the aircraft for extended periods of time beyond what is scheduled for routine maintenance cycles. Because current cleaning methods do not sterilize the water system, microbes are left within the system and can continue to grow as long as conditions permit. Once biologically contaminated, microbial growth may occur for the life of the system. To reduce the time the aircraft is out of operation, the buildup of biofilms and scale often goes ignored until a problem is detected (e.g., clog, leak, or reduced water quality) by which point, extended maintenance can be required.

A need exists for a method and apparatus to clean and sterilize fluid system components in-situ as part of a regular maintenance cycle in order to reduce long-term repair requirements, increase efficiency, and improve water quality.

### SUMMARY

In one aspect, a self-cleaning component assembly includes a fluid containment vessel, an ultrasonic transducer fixed to a wall of the fluid containment vessel, and a germicidal light source fixed to the component such that germicidal light is directed to an internal cavity of the fluid containment vessel.

In another aspect, a method of cleaning fluid containment components includes applying ultrasonic vibrations to a fluid containment vessel in situ to break up and dislodge material buildup on surfaces in a cavity of the vessel, flushing the cavity of the vessel with fluid to remove material buildup, and applying germicidal light to the cavity of the vessel using a germicidal light source fixed through a wall of the fluid containment vessel.

In yet another aspect, an automated component cleaning assembly includes a fluid containment vessel, an ultrasonic transducer fixed to a wall of the fluid containment vessel, a germicidal light source fixed to the component such that germicidal light is directed to an internal cavity of the fluid containment vessel, and a controller connected to the ultrasonic transducer and germicidal light source and configured to turn on and off power to the ultrasonic transducer and the germicidal light source.

The present summary is provided only by way of example, and not limitation. Other aspects of the present disclosure will be appreciated in view of the entirety of the present disclosure, including the entire text, claims and accompanying figures.

### BRIEF DESCRIPTION OF THE DRAWINGS

FIG. 1 is schematic cross-sectional view of one embodiment of a self-cleaning component assembly.
FIG. 2 is a perspective view of another embodiment of the self-cleaning component assembly.
FIG. 3 is a method flow chart for cleaning fluid system components.

While the above-identified figures set forth embodiments of the present invention, other embodiments are also contemplated, as noted in the discussion. In all cases, this disclosure presents the invention by way of representation and not limitation. It should be understood that numerous other modifications and embodiments can be devised by those skilled in the art, which fall within the scope of the invention as defined by the claims.. The figures may not be drawn to scale, and applications and embodiments of the present invention may include features, steps and/or components not specifically shown in the drawings.

### DETAILED DESCRIPTION

Ultrasonic transducers, capable of producing ultrasonic vibrations, and germicidal light sources, such as UV-C LEDs, can be permanently fixed to fluid transport and storage components to remove mineral scale and microbial contamination from the components as part of a self-cleaning, routine maintenance cycle, thereby reducing a need for time-intensive hand cleaning, chemical treatment, or replacement of parts as required to address clogging, corrosion, reduction in performance, and contamination of water supplies.

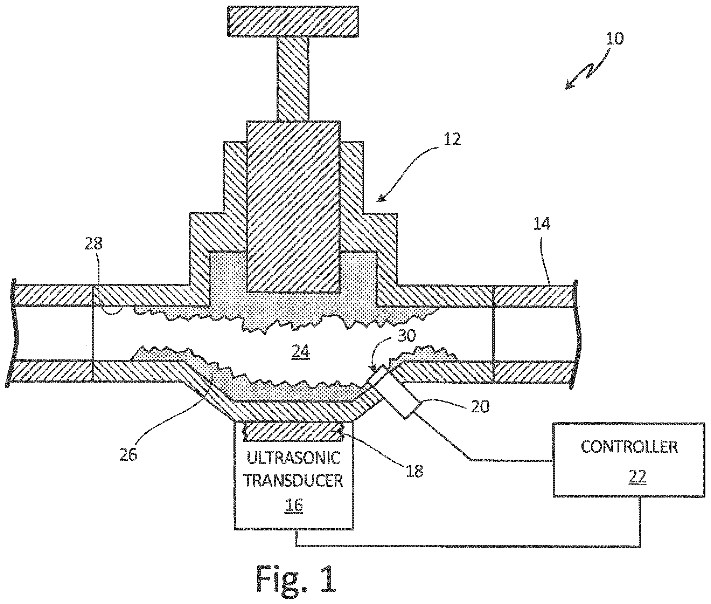

FIG. 1 is a schematic cross-sectional view of one embodiment of self-cleaning component assembly 10. FIG. 1 depicts self-cleaning component assembly 10 as installed in valve 12 of a fluid delivery system. As will be discussed further and will be understood by one of ordinary skill in the art, self-cleaning component assembly 10 can be installed in a variety of components, including, but not limited to valves, storage containment vessels (e.g., water heaters, storage tanks, etc.), pipes, and other fluid delivery components. Self-cleaning component assembly 10 can include valve 12, pipe 14, ultrasonic transducer 16, mount 18, germicidal light source 20, and controller 22. Valve 12 can be connected to pipe 14 or any other fluid containment vessel. Ultrasonic transducer 16 can be attached to valve 12 by mount 18. Germicidal light source can be attached to valve 12 such that germicidal light can be directed to internal cavity 24 of valve 12. One or more controllers 22 can be electrically connected to ultrasonic transducer 16 and germicidal light 20 to control operation of ultrasonic transducer 16 and germicidal light 20.

Material 26, which can include biofilm and mineral scale, can build up in fluid valve 12 as well as other fluid system components and can cause clogging, corrosion, loss of efficiency, and fluid contamination among other issues. Ultrasonic transducer 16 and germicidal light source 20 can be installed in one or more areas susceptible to material buildup 26 to partially or fully remove scale and biofilm present on walls 28 and other surfaces in internal cavity 24 as well as microbial contamination present in the fluid contained by valve 12. Generally, material buildup can occur in more complex geometries (as opposed to smooth or vertical surfaces) and areas where there is surface corrosion. In general, scale can more strongly adhere to surfaces than biofilm and, therefore, increased ultrasonic agitation can be required in areas susceptible to scaling. In addition to removing scale and biofilm from surfaces, ultrasonic agitation can break up microbial colonies, resulting in more effective germicidal light treatment.

Ultrasonic transducer 16 can produce high frequency sound waves, generally between 20 kilohertz (kHz) and 80 kHz, to agitate fluid and clean internal cavity 24 through the process of cavitation. Ultrasonic transducer 16 can be any type of transducer device as known in the art for ultrasonic cleaning, including but not limited to, a piezoelectric transducer, and can operate in a manner consistent with ultrasonic transducers used for cleaning. The resonant frequency and power at which ultrasonic transducer 16 is operated can be varied according to the application. Generally, the resonant frequency of the embodiments disclosed in the present application can be between 40 kHz and 60 kHz. In some embodiments, the resonant frequency can be closer to 50 kHz. Generally, ultrasonic transducer 16 can be powered at between 40 watts (W) and 60 W, however, it will be understood by one of ordinary skill in the art that power can also be varied according to application as needed to effectively clean components. Self-cleaning component assemblies 10 disclosed in the present application can effectively clean components of a potable water system during a routine aircraft maintenance cycle. The application of ultrasonic cleaning can generally be completed in 10-30 minutes, however the duration of ultrasonic cleaning can vary depending on the extent of material buildup 26 present, frequency and output of ultrasonic transducer 16, and the volume of internal cavity 24.

Ultrasonic transducer 16 can be fixed to an external surface of valve wall 28 by mount 18. Mount 18 can be permanently fixed to valve 12 (e.g., via weld or epoxy adhesive) or removably fixed to valve 12 (e.g., via threaded fastener). Ultrasonic transducer 16 can likewise be permanently fixed and/or removably fixed to mount 18. In the embodiment shown in FIG. 1, mount 18 includes a bolt welded to valve 12 to which ultrasonic transducer 16 is threadedly fastened and permanently fixed using an epoxy adhesive. In other embodiments, ultrasonic transducer 16 can be directly fixed to wall 28 of valve 12. In all embodiments, ultrasonic transducer 16 remains in place during both a cleaning cycle and normal operation of the components (i.e., ultrasonic transducer 16 remains installed on valve 12 when the aircraft is in flight and when the aircraft is grounded). It will be understood by one of ordinary skill in the art that the means by which ultrasonic transducer 16 is attached to valve 12 can be of any type capable of securely fastening ultrasonic transducer 16 to valve 12 during all operations. Threaded and similar types of fasteners can allow for easier replacement of ultrasonic transducer 16 in the event that ultrasonic transducer 16 malfunctions or needs replacement.

One or more germicidal light sources 20 can be attached to valve 12 such that germicidal light can be directed to internal cavity 24 of valve 12. As shown in FIG. 1, germicidal light source 20 can extend through wall 28 of valve 12 to internal cavity 24. Germicidal light source 20 can be sealed and fixed within wall 28 by any means known in the art. In the embodiment shown in FIG. 1, valve 12 includes a threaded fitting in wall 28 in which germicidal light source 20 can be threadedly fastened. Germicidal light source 20 can further be fixed to wall 28 with an epoxy adhesive. One or more seals (not shown) can be used to prevent fluid leakage from valve 12 at the location of germicidal light source 20. Transmission sheet 30 can cover an opening in wall 28 through which germicidal light is directed. Transmission sheet 30 can be a quartz transmission sheet or other suitable protective covering that allows the transmission of germicidal light. Transmission sheet 30 can separate germicidal light source 20 from the fluid within internal cavity 24 and thereby protect germicidal light source 20.

Germicidal light source 20 can produce short wave UV-C light that can kill or render inactive bacteria, viruses, and other pathogens that can contaminate water supplies. Regular use of germicidal light sterilization during routine maintenance cycles or during operation of the water supply system can improve water quality and reduce the potential for microbial induced corrosion. Germicidal light source 20 can be a UV-C LED or other suitable UV-C light source. It will be understood by one of ordinary skill in the art that germicidal light sources 20 can be selected as appropriate for varying applications and available power supply. For example, in one embodiment, germicidal light source 20 can be a UV-C LED with peak emission wavelength of 255 nm and power output of 1-2 mW. In other embodiments, germicidal light sources 20 having an increased power output can be used to reduce the time required for cleaning. Germicidal light source 20 can be positioned within valve 12 to optimize an amount of light directed into internal cavity 24. Multiple germicidal light sources 20 can be used to improve decontamination. Additionally, multiple germicidal light sources 20 or germicidal light sources 20 with greater power output can be used for larger components, components with more complex geometries, and water sources having higher mineral content or microbial contamination or particularly resilient strains of bacteria.

Ultrasonic transducer 16 and germicidal light 20 can be connected to one or more controllers 22 to supply power to ultrasonic transducer 16 and germicidal light 20. Controller 22 can be programmable to allow for automated cleaning or can require manual input. In some embodiments, controller 22 can also be used to operate the fluid supply system.

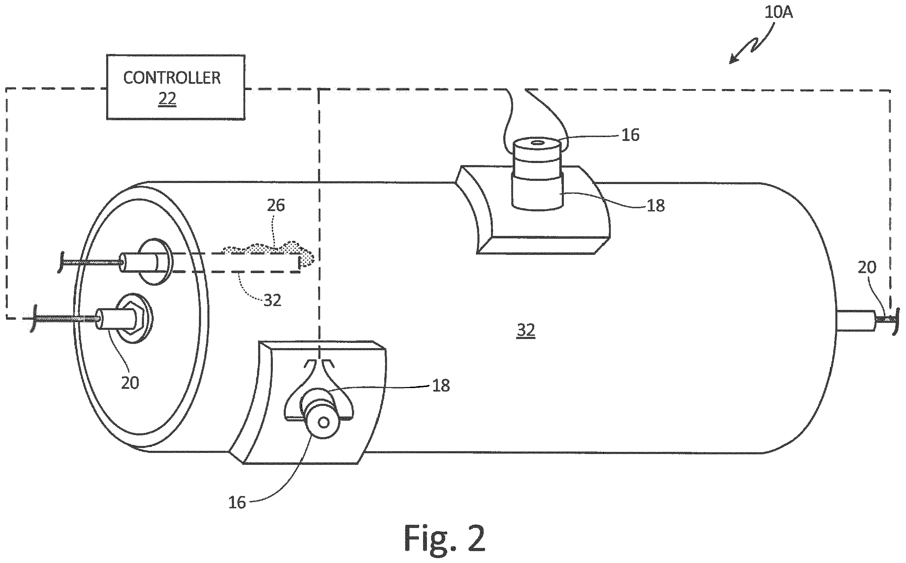

FIG. 2 is a perspective view of self-cleaning component assembly 10A as installed in water heater tank 32 of a fluid delivery system. As shown in FIG. 2, self-cleaning component assembly 10A can include tank 32, multiple ultrasonic transducers 16, mounts 18, and germicidal light sources 20, controller 22, and probe 34. Ultrasonic transducers 16 and germicidal light sources 20 can be installed on tank 32 as disclosed above in relation to FIG. 1. In the embodiment shown in FIG. 2, ultrasonic transducers 16 are spaced apart from one another. The spacing shown is not intended to indicate an optimal arrangement or limit the placement of ultrasonic transducers 16. It will be understood by one of ordinary skill in the art that optimal placement of ultrasonic transducers 16 can vary depending on the size, internal geometry, and material buildup 26 in the component along walls and/or other structures within the internal cavity. For instance, self-cleaning component assembly 10A can include probe 34 (e.g., conductivity probe), which can be susceptible to material buildup 26. To ensure effective removal of material buildup 26 on probe 34, ultrasonic transducer 16 can be positioned with closer proximity to probe 34.

As shown in FIG. 2, germicidal light sources 20 can be attached to tank 32 such that germicidal light can be directed to an internal cavity (not shown) of tank 32 along a length of tank 32. Again, the positioning of germicidal light sources 20 shown is not intended to indicate an optimal arrangement or limit the placement of germicidal light sources 20. It will be understood by one of ordinary skill in the art that optimal placement of germicidal light sources 20 can vary depending on the volume of the internal cavity and complexity of the internal geometry of the component.

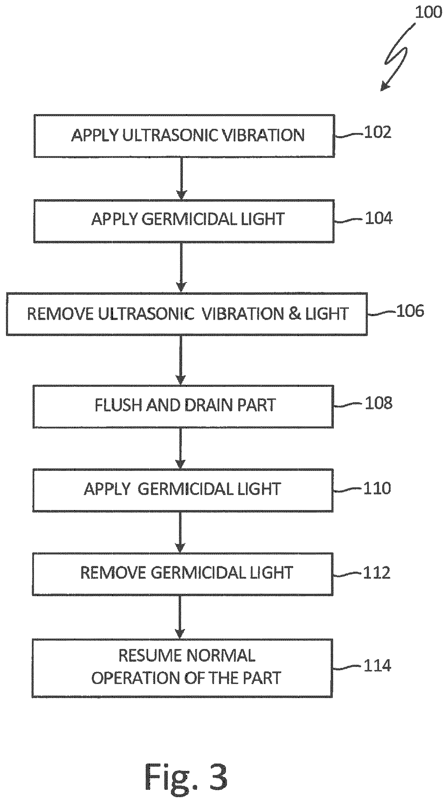

FIG. 3 is a flow chart of method 100 of operating self-cleaning component assembly 10. Self-cleaning component assembly 10 can be used to remove scale, biofilm, and microbial contamination within components in a fluid supply system. Self-cleaning component assembly 10 is particularly suited to cleaning a potable water supply system on an aircraft during a routine maintenance cycle and will be described in that manner. However, it will be understood by one of ordinary skill in the art that self-cleaning component assembly 10 can be adapted for use in a wide variety of fluid delivery and storage systems.

Self-cleaning component assembly 10 can be used in a purge/cleaning cycle of an aircraft potable water supply system during routine maintenance cycles when the aircraft is grounded. Ultrasonic transducer 16 can be turned on to agitate water within the water supply system and break up and dislodge scale and biofilm from component surfaces (e.g., wall 28) by cavitation (step 102). The application of ultrasonic cleaning can generally be completed in 10-30 minutes, however the duration of ultrasonic cleaning can vary depending on the extent of material buildup 26 present, frequency and output of ultrasonic transducer 20, and the volume of internal cavity 24. Germicidal light can be applied to internal cavity 24 during the process of ultrasonic cleaning (step 104). Once ultrasonic cleaning is complete, power to both ultrasonic transducer 16 and germicidal light source 20 can be shut off (step 106) and components can be flushed with water and drained (step 108) to carry away material 26 that has been removed from surfaces within internal cavity 24. Once the water supply system has been flushed and drained, components can be refilled with fresh water. Germicidal light can then again be applied to internal cavity 24 (step 110) to further decontaminate surfaces and microbial contamination in the water. The decontamination process can range from minutes to hours depending on the strength of germicidal light source 20. Use of a low power UV-C LED, as described above, can increase the time required for decontamination. However, because of the low power requirement, germicidal light source 20 can be powered using normal aircraft power, which can allow for continued use during flight as needed. The ability to extend the use of germicidal light during flight can be particularly beneficial when water quality is poor, which can be the case when operating in certain countries. In other cases, germicidal light source 20 can be shut off during the maintenance cycle, following a specified run time determined to provide sufficient decontamination of the component (step 112). Germicidal light source 20 can then remain off when the aircraft is in flight and use of the potable water system is resumed (step 114).

Self-cleaning component assembly 10, including fixed ultrasonic transducer 16 and germicidal light source 20, can reduce costly downtime generally required to repair, replace, and clean fluid supply system components. Self-cleaning component assembly 10 can be implemented into fluid supply systems and operated during routine maintenance cycles to keep components clean and in good operating condition.

### Discussion of Possible Embodiments

The following are non-exclusive descriptions of possible embodiments of the present invention.

A self-cleaning component assembly includes a fluid containment vessel, an ultrasonic transducer fixed to a wall of the fluid containment vessel, and a germicidal light source fixed to the component such that germicidal light is directed to an internal cavity of the fluid containment vessel.

The self-cleaning component assembly of the preceding paragraph can optionally include, additionally and/or alternatively, any one or more of the following features, configurations and/or additional components:
A further embodiment of the foregoing self-cleaning component assembly, wherein the germicidal light source can extend through the wall of the fluid containment vessel to the internal cavity.

A further embodiment of any of the foregoing self-cleaning component assemblies, wherein the ultrasonic transducer can be fixed to a mount on an external surface of the fluid containment vessel.

A further embodiment of any of the foregoing self-cleaning component assemblies, wherein the germicidal light source can be a UV-C LED.

A further embodiment of any of the foregoing self-cleaning component assemblies, wherein the ultrasonic transducer can have a resonant frequency between 40 and 60 kHz.

A further embodiment of any of the foregoing self-cleaning component assemblies can further include an additional ultrasonic transducer fixed to the fluid containment vessel.

A further embodiment of any of the foregoing self-cleaning component assemblies can further include an additional germicidal light source fixed to the fluid containment vessel.

A further embodiment of any of the foregoing self-cleaning component assemblies can further include a quartz transmission cover fixed and sealed to the wall at the internal cavity and positioned to separate the germicidal light source from a fluid in the internal cavity.

A further embodiment of any of the foregoing self-cleaning component assemblies can further include a seal between the internal cavity of the fluid containment vessel and the germicidal light source.

A further embodiment of any of the foregoing self-cleaning component a assemblies, wherein the fluid containment vessel can be selected from a group consisting of a tank, a pipe, and a valve.

A method of cleaning fluid containment components includes applying ultrasonic vibrations to a fluid containment vessel in situ to break up and dislodge material buildup on surfaces in a cavity of the vessel, flushing the cavity of the vessel with fluid to remove material buildup, and applying germicidal light to the cavity of the vessel using a germicidal light source fixed through a wall of the fluid containment vessel.

The method of the preceding paragraph can optionally include, additionally and/or alternatively, any one or more of the following steps, features, configurations and/or additional components:
A further embodiment of the foregoing method, wherein the steps of applying ultrasonic vibrations and applying germicidal light can be conducted simultaneously.

A further embodiment of any of the foregoing methods, wherein the step of applying germicidal light can be conducted after the step of flushing the cavity.

A further embodiment of any of the foregoing methods can further include halting operation of the ultrasonic transducer and germicidal light source, and commencing normal operation of the fluid supply system. The ultrasonic transducer and germicidal light source can remain fixed to the fluid containment vessel during normal operation of the fluid supply system.

A further embodiment of any of the foregoing methods, wherein the steps of applying ultrasonic vibrations, flushing the cavity, and applying germicidal light can be automated.

An automated component cleaning assembly includes a fluid containment vessel, an ultrasonic transducer fixed to a wall of the fluid containment vessel, a germicidal light source fixed to the component such that germicidal light is directed to an internal cavity of the fluid containment vessel, and a controller connected to the ultrasonic transducer and germicidal light source and configured to turn on and off power to the ultrasonic transducer and the germicidal light source.

The automated component cleaning assembly of the preceding paragraph can optionally include, additionally and/or alternatively, any one or more of the following features, configurations and/or additional components:

A further embodiment of the foregoing automated component cleaning assembly, wherein the ultrasonic transducer can be fixed to a mount on an external surface of the fluid containment vessel and wherein the germicidal light source can extend through the wall of the fluid containment vessel to the internal cavity of the fluid containment vessel.

A further embodiment of any the foregoing automated component cleaning assemblies, wherein the ultrasonic transducer can have a resonance frequency between 40 and 60 kHz.

A further embodiment of any the foregoing automated component cleaning assemblies can further include an additional ultrasonic transducer fixed to the fluid containment vessel.

A further embodiment of any the foregoing automated component cleaning assemblies can further include an additional germicidal light source fixed to the fluid containment vessel.

### Summation

Any relative terms or terms of degree used herein, such as "substantially", "essentially", "generally", "approximately" and the like, should be interpreted in accordance with and subject to any applicable definitions or limits expressly stated herein. In all instances, any relative terms or terms of degree used herein should be interpreted to broadly encompass any relevant disclosed embodiments as well as such ranges or variations as would be understood by a person of ordinary skill in the art in view of the entirety of the present disclosure, such as to encompass ordinary manufacturing tolerance variations, incidental alignment variations, alignment or shape variations induced by thermal, rotational or vibrational operational conditions, and the like.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the scope thereof. Therefore, it is intended that the invention not be limited to the particular embodiment(s) disclosed, but that the invention will include all embodiments falling within the scope of the appended claims.

## Claims

1. A self-cleaning component assembly comprising:
a fluid containment vessel (14);
an ultrasonic transducer (16) fixed to a wall of the fluid containment vessel; and
a germicidal light source (20) fixed to the component such that germicidal light is directed to an internal cavity (24) of the fluid containment vessel.

2. The self-cleaning component assembly of claim 1, wherein the germicidal light source extends through the wall of the fluid containment vessel to the internal cavity.

3. The self-cleaning component assembly of claim 1 or 2, wherein the ultrasonic transducer is fixed to a mount (18) on an external surface of the fluid containment vessel.

4. The self-cleaning component assembly of any preceding claim, wherein the germicidal light source is a UV-C LED.

5. The self-cleaning component of any preceding claim and further comprising:
an additional ultrasonic transducer fixed to the fluid containment vessel; and/or further comprising:
an additional germicidal light source fixed to the fluid containment vessel.

6. The self-cleaning component of any preceding claim and further comprising:
a quartz transmission cover fixed and sealed to the wall at the internal cavity and positioned to separate the germicidal light source from a fluid in the internal cavity.

7. The self-cleaning component of any preceding claim and further comprising:
a seal between the internal cavity of the fluid containment vessel and the germicidal light source.

8. The self-cleaning component assembly of any preceding claim, wherein the fluid containment vessel is selected from a group consisting of a tank, a pipe, and a valve.

9. A method of cleaning fluid containment components in a fluid supply system comprising:
applying ultrasonic vibrations to a fluid containment vessel in situ to break up and dislodge material buildup on surfaces in a cavity of the vessel, wherein ultrasonic vibrations are applied using an ultrasonic transducer fixed to a wall of a fluid containment vessel;
flushing the cavity of the vessel with fluid to remove material buildup; and
applying germicidal light to the cavity of the vessel with a germicidal light source fixed through a wall of the fluid containment vessel.

10. The method of claim 9, wherein the steps of applying ultrasonic vibrations and applying germicidal light are conducted simultaneously.

11. The method of claim 9 or 10, wherein the step of applying germicidal light is conducted after the step of flushing the cavity.

12. The method of claim 9, 10 or 11 and further comprising:
halting operation of the ultrasonic transducer and germicidal light source; and
commencing normal operation of the fluid supply system;
wherein the ultrasonic transducer and germicidal light source remain fixed to the fluid containment vessel during normal operation of the fluid supply system.

13. An automated component cleaning assembly comprising:
a fluid containment vessel (14);
an ultrasonic transducer (16) fixed to a wall of the fluid containment vessel;
a germicidal light source (20) fixed to the component such that germicidal light is directed to an internal cavity of the fluid containment vessel; and
a controller connected to the ultrasonic transducer and germicidal light source and configured to turn on and off power to the ultrasonic transducer and the germicidal light source.

14. The automated component cleaning assembly of claim 13, wherein the ultrasonic transducer is fixed to a mount on an external surface of the fluid containment vessel and wherein the germicidal light source extends through the wall of the fluid containment vessel to the internal cavity of the fluid containment vessel.

15. The automated component cleaning assembly of claim 13 or 14 and further comprising:
an additional ultrasonic transducer fixed to the fluid containment vessel; and/or further comprising:
an additional germicidal light source fixed to the fluid containment vessel.
